# EUROPEAN PATENT APPLICATION

(11) **EP 1 775 300 A1**
(43) Date of publication of application: **18.04.2007**
(21) Application number: 05766322.1
(22) Date of filing: 15.07.2005
(51) Int. Cl.: C07F 7/18, A61K 31/047, A61K 31/22, A61P 33/06, A61P 35/00

(54) **PROCESS FOR PRODUCTION OF POLYSUBSTITUTED CYCLOBUTANES AND POLYSUBSTITUTED CYCLOBUTENES**

(30) Priority: 22.07.2004 JP 2004010408
(71) Applicant: National University Corporation Tohoku Unversity, Sendai Miyagi 980577 (JP)
(72) Inventor: TAKASU, Kiyosei, Sendai-shi, Miyagi 982-0012 (JP); IHARA, Masataka, Sendai-shi, Miyagi 982-0021 (JP); INENAGA, Kazato, Sendai-shi, Miyagi 982-0801 (JP)
(74) Representative: Seiffert, Klaus
(86) International application number: PCT/JP2005/013177
(87) International publication number: WO 2006/009119

(57) **Abstract**

The prior art required specialized substrates or reaction conditions to be used to manufacture polysubstituted cyclobutane compounds and polysubstituted cyclobutene compounds, and the method had poor generality. The type or quantity of the catalyst or solvent used was also problematic in the industrial manufacture of polysubstituted cyclobutane compounds. The present invention provides a method for manufacturing a polysubstituted cyclobutane compound with high stereoselectivity that has low environmental load (is ecologically advantageous) and is applicable to industrial manufacturing from the standpoint of operation, substrate generality, catalyst, solvent, and efficiency. A polysubstituted cyclobutane, a cyclobutene, and a bicyclo[4.2.0]octane compound can be manufactured efficiently, stereoselectively, and in an ecologically advantageous manner by causing a Brønsted acid to act on a mixture of an enol ether compound or 2-siloxydiene compound with an alkene or alkyne compound in which a carbonyl group is substituted at the 1-position in a non-aqueous solvent or without a solvent.

## Description

### TECHNICAL FIELD

An object of the present invention is to provide a polysubstituted cyclobutane compound [Chemical Formula (3)] having substituents at consecutive 1,2-positions on a four-membered ring, to provide a simple and highly general method for manufacturing a polysubstituted cyclobutene compound [Chemical Formula (12)] and a polysubstituted bicyclo[4.2.0]octane compound [Chemical Formula (15)], and to provide compounds indicated by Chemical Formulae (3), (12), and (15) obtained by the manufacturing method.

### BACKGROUND ART

Cyclobutane and other four-membered ring compounds provide specific conformations that originate from ring strain, and are therefore have high potential value as structural elements of pharmaceutical products. Cyclobutanes and other four-membered ring compounds also exhibit unique reactive properties, and can therefore be utilized as synthons. For example, numerous compounds are obtained as natural extracts that contain cyclobutane [1], cyclobutene [2], and/or bicyclo[4.2.0]octane [3] as component structures. Compounds 1 and 2 have strong antimicrobial effects against fungi or bacteria, and therefore have potential applications as antibiotics and other pharmaceutical products. Compound 3 is known to contribute to silver leaf disease in plants, and can therefore be utilized as a pesticide for exterminating weeds and the like, or applied as an agent for preventing silver leaf disease through the creation of a structural analog of compound 3 (to function as a receptor antagonist). Compounds 4 and 5 are isolated as sex pheromones (attractants) for Anthonomus grandis grandis and Pseudococcidae, respectively. These pests inflict serious damage to farm crops, and the cost of agricultural damage caused by Pseudococcidae in particular is reported to be 750 million dollars per year. Compounds 4 and 5 can be directly utilized as industrial pesticides (insecticides) when synthesizable in large quantities. Compound 6 is recognized as having cytotoxic effects against cancer cells, and the application thereof in anticancer drugs can be anticipated. Various other biologically active substances are known that contain cyclobutane, cyclobutene, and/or bicyclo[4.2.0]octane.

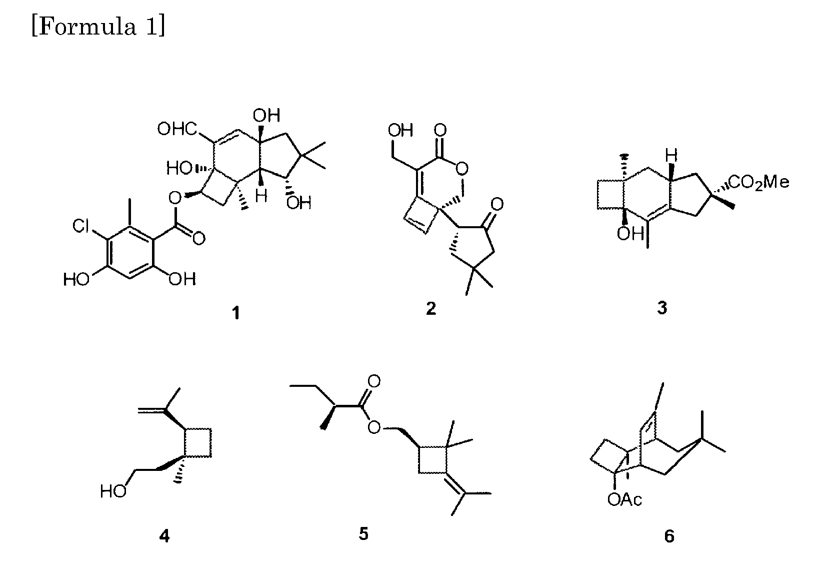

However, cyclobutane species are hardly ever used when actual industrial utilization is emphasized. Almost of the methods for synthesizing polysubstituted cyclobutane compounds reported in the past had complex multiple steps, or involved cycloaddition reactions using light that had little generality, or thermal cycloaddition reactions (Patent References 1 and 2) in which the substrate was a ketene, a polyalkoxycarbonyl olefin, or another extremely electron-deficient alkene or alkyne, or a polyalkoxy olefin or other extremely electron-rich alkene. Accordingly, these methods could not be applied to commonly used substrates and had little usefulness in synthesis.

Synthesis methods that are exceptions to the one mentioned above are ones in which a Lewis acid catalyst is reacted with an α,β-unsaturated carbonyl compound and a somewhat atypical substrate such as a thioether, a thioalkyne, or a selenoether (Non-patent References 1 and 2). These methods are considered to have a great deal of potential compared to the aforementioned thermal cycloaddition reaction in terms of reaction diversity and other characteristics. However, because the reaction is limited to a specialized substrate, these methods have a large number of drawbacks relating to industrial utilization in the pharmaceutical and materials fields. A method for manufacturing a polysubstituted cyclobutene compound is already known that uses an enol ether compound and an alkyne compound as starting materials (Non-patent Reference 3), but one or more equivalent amounts of titanium tetrachloride or another reagent that is difficult to handle industrially must be used in this case. Specifically, this method is disadvantageous from the standpoint of environmentally advantageous chemistry, and is considered difficult to utilize industrially.

The inventors recently reported a synthesis method whereby ethyl aluminum dichloride or diethyl aluminum chloride is reacted with a silyl enol ether and an α,β-unsaturated carbonyl compound (Non-patent References 4 and 5). The inventors also reported a method for synthesizing a polysubstituted bicyclo[4.2.0]octane compound by reacting excess quantities of an α,β-unsaturated carbonyl compound and ethyl aluminum dichloride with a 2-siloxy diene (Non-patent Reference 6). The catalyst used in these methods is extremely difficult to handle, as the catalyst is known to be ignitable or become inactive in the presence of minute quantities of water included in the atmosphere or the reaction solvent. These methods also require a quantity of 20 mol% or more of the catalyst with respect to the starting material, which makes these methods impractical for industrial application due to the inevitable increase in cost and danger. The publicly known methods also have drawbacks in that the reaction solvents used therein are methylene chloride, dichloroethane, and other halogenated solvents, and the use of large quantities of these solvents therefore causes environmental contamination. Specifically, it is practically impossible to provide the industrially required quantity of the polysubstituted cyclobutane, the polysubstituted cyclobutene, or the polysubstituted bicyclo[4.2.0]octane compound by these methods. The inventors then conducted a concentrated investigation, but were unable to discover an industrially adequate manufacturing method insofar as a publicly known Lewis acid catalyst was used.

A polysubstituted cyclobutane compound having an ether substituent and a side chain having a carbonyl group on a four-membered ring is a highly asymmetrical compound that is highly reactive, and there are therefore no known simple methods for synthesizing this compound that have high generality. However, there are numerous groups of this type of compound in the design/synthesis or derivatization of desired functional molecules or pharmaceutical products. There is therefore a need to develop a method for manufacturing a polysubstituted cyclobutane compound that is economical and ecologically advantageous.

[Patent Reference 1] WO 9852930
[Patent Reference 2] EP 893427
[Non-patent Reference 1] Journal of Organic Chemistry, 1992, Vol. 57, pp. 5610-5619.
[Non-patent Reference 2] Journal of the American Chemical Society, 1992, Vol. 114, pp. 8869-8885.
[Non-patent Reference 3] Tetrahedron Letters, 1988, Vol. 29, No. 49, pp. 6443-6446.
[Non-patent Reference 4] Journal of Organic Chemistry, 2004, Vol. 69, No. 2, pp. 517-521.
[Non-patent Reference 5] Tetrahedron, 2004, Vol. 60, No. 9, pp. 2071-2078.
[Non-patent Reference 6] Journal of the American Chemical Society, 2004, Vol. 126, No. 5, pp. 1352-1353.

### DISCLOSURE OF THE INVENTION

### [Problems the Invention Is Intended To Solve]

As described above, the prior art required the use of specialized substrates or reaction conditions in order to manufacture a polysubstituted cyclobutane compound and a polysubstituted cyclobutene compound, and the method of the prior art had poor generality and low usefulness in synthesis. The conventional method also had severe drawbacks relating to the stability of the catalyst, the reaction cost, and environmental contamination by solvents or waste products, and was impossible to apply to industrial manufacturing.

An object of the present invention is to provide a method for manufacturing a polysubstituted cyclobutane compound that has low environmental load and is applicable to industrial manufacturing from the standpoint of operation, substrate generality, catalyst, solvent, and efficiency. Specific requirements are that the amount of catalyst used is in a mole ratio of approximately 1% or less of the reaction starting materials (this requirement was a minimum of 20% or higher in the prior art), that the reaction need not be performed in an environment that is strictly devoid of water or air (a completely sealed dry vessel was required in the prior art), and that the reaction can be performed without using a halogenated solvent (methylene chloride or dichloroethane, for example) that causes environmental contamination. Specifically, the prior art relied on a method that was extremely inefficient even for synthesizing 100-milligram units of a polysubstituted cyclobutane or a cyclobutene compound, and a method is now needed for safely manufacturing these compounds at a practical level at low cost.

The present invention provides a method for manufacturing a polysubstituted cyclobutane compound indicated by Chemical Formula (3): 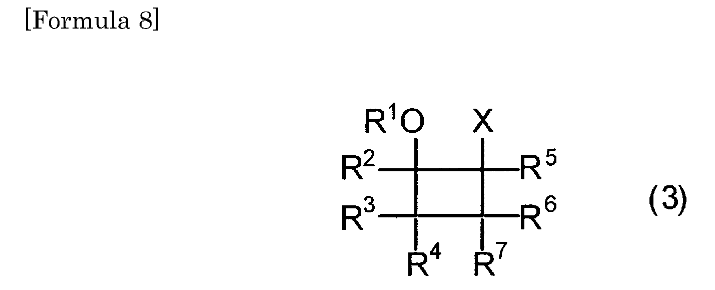 (wherein X and the substituents R¹ through R⁷ are the same as below) by reacting an enol ether compound indicated by Chemical Formula (1): 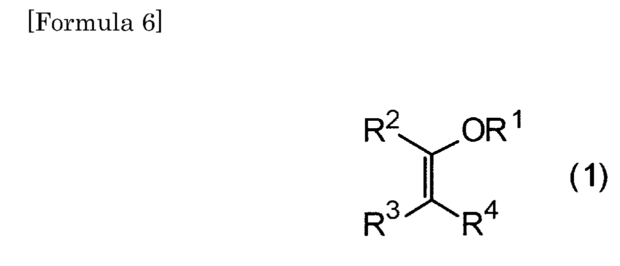 (wherein R¹ is an optionally substituted alkyl group, an optionally substituted aryl group, an optionally substituted heterocyclic group, or a silyl group; R², R³, and R⁴ are each independently a hydrogen atom, a halogen atom, an optionally substituted alkyl group, an optionally substituted aryl group, an optionally substituted heterocyclic group, or an optionally substituted alkyl alkoxy group, and may be the same or different; and the substituents of R¹ through R⁴ may be bonded to each other); and
an alkene compound in which a carbonyl group is substituted at the 1-position, wherein the alkene compound is indicated by Chemical Formula (2): 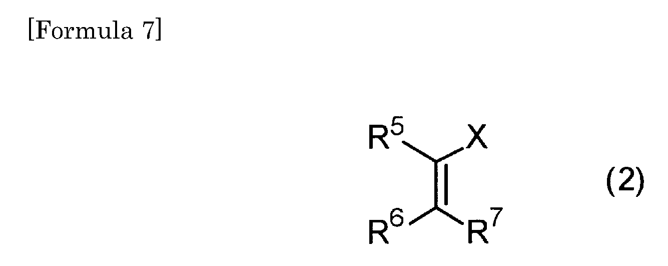 (wherein X is an ester carbonyl group, an amide carbonyl group, a ketocarbonyl group, or an aldehyde group; R⁵, R⁶, and R⁷ are each independently a hydrogen atom, a halogen atom, an optionally substituted alkyl group, an optionally substituted aryl group, or an optionally substituted heterocyclic group, and may be the same or different; and the substituents of X and R⁵ through R⁷ may be bonded to each other); wherein the method for manufacturing a polysubstituted cyclobutane compound is characterized in that a Brønsted acid catalyst is made to act in a non-aqueous solvent. The Brønsted acid is preferably a trifluoromethane sulfonimide, a pentafluorobenzene sulfonimide, bis(pentafluoroethyl)phosphoric acid, or a strong acid such as one having a substituted polyfluoroalkyl sulfonic acid group indicated by Chemical Formula (6): 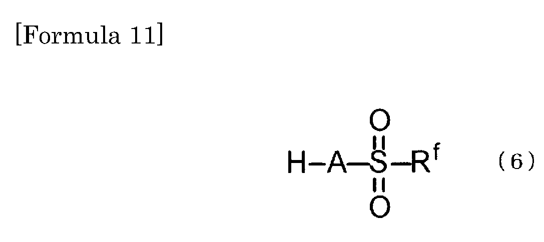 (wherein A is a nitrogen atom, an oxygen atom, a carbon atom, a sulfur atom, or a phosphorus atom that has a plurality of substituents or is unsubstituted as chemically allowed; and R^{f} is a heterocyclic group, an aryl group, or an alkyl group in which two or more fluorine atoms are substituted). Examples of this type of acid include bis(trifluoromethane)sulfonimide, bis(pentafluoroethane)sulfonimide, bis(pentafluorobenzene)sulfonimide, N-pentafluorobenzene sulfonyl-N-trifluoromethane sulfonimide, N-trifluoromethane sulfonyl-N-trifluoromethane sulfonimide, trifluoromethane sulfonic acid, pentafluoroethane sulfonic acid, tris(trifluoromethanesulfonyl)methane, tris(pentafluorobenzenesulfonyl)methane, bis(pentafluorobenzenesulfonyl)torfluoromethane (*3) sulfonyl methane, and the like. However, these compounds are merely examples, and the present invention is not limited by the abovementioned compounds.

The present invention also provides a method for selectively synthesizing a stereoisomer indicated by Chemical Formula (10) that is synthesizable by the abovementioned manufacturing method, in which an oxygen substituent and a substituent X on a four-membered ring are in a trans relationship 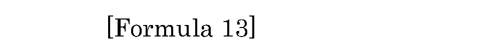 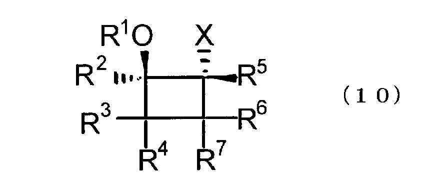 (wherein X and the substituents of R¹ through R⁷ are the same as above). In this case, the substituent X of the alkene compound as the starting material is preferably an ester carbonyl group, and examples thereof include an alkoxy carbonyl group and a polyhaloalkoxy carbonyl group. The enol ether compound as the starting material is also preferably a silyl enol ether, and examples thereof include enol ethers in which a triisopropyl silyl group or a tert-butyldimethyl silyl group is substituted.

The present invention also provides a polysubstituted cyclobutane compound that is synthesizable by the method of the present invention described above.

Another aspect of the present invention provides a method for manufacturing a polysubstituted cyclobutene compound indicated by Chemical Formula (12): 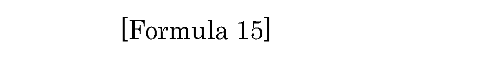 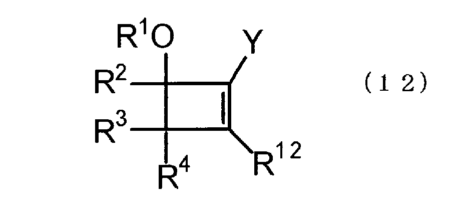 (wherein Y, R¹ through R⁴, and R¹² are the same as below) by reacting an enol ether compound indicated by Chemical Formula (1) and an alkyne compound indicated by Chemical Formula (11): 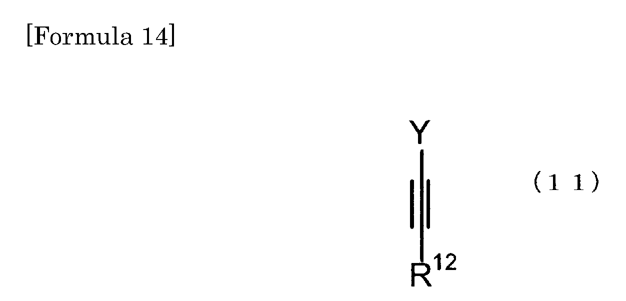 (wherein Y is an ester carbonyl group, an amide carbonyl group, a ketocarbonyl group, an aldehyde group, or a nitrile group; and R¹² is a hydrogen atom, a halogen atom, an optionally substituted alkyl group, an optionally substituted aryl group, or an optionally substituted heterocyclic group); wherein the method for manufacturing a polysubstituted cyclobutene compound is characterized in that a Brønsted acid catalyst is made to act in a non-aqueous solvent. The Brønsted acid is preferably a trifluoromethane sulfonimide, a pentafluorobenzene sulfonimide, bis(pentafluoroethyl)phosphoric acid, or a strong acid such as one having a substituted polyfluoroalkyl sulfonic acid group indicated by Chemical Formula (6). Examples of this type of acid include bis(trifluoromethane)sulfonimide, bis(pentafluoroethane)sulfonimide, bis(pentafluorobenzene)sulfonimide, N-pentafluorobenzene sulfonyl-N-trifluoromethane sulfonimide, N-trifluoromethane sulfonyl-N-trifluoromethane sulfonimide, trifluoromethane sulfonic acid, pentafluoroethane sulfonic acid, tris(trifluoromethanesulfonyl)methane, tris(pentafluorobenzenesulfonyl)methane, bis(pentafluorobenzenesulfonyl)(trifluoromethane sulfonyl)methane, and the like. However, these compounds are merely examples, and the present invention is not limited by the abovementioned compounds.

In the manufacturing method described above, the substituent Y of the alkyne compound as the starting material is preferably an ester carbonyl group, and examples thereof include an alkoxy carbonyl group and a polyhaloalkoxy carbonyl group. The enol ether compound as the starting material is also preferably a silyl enol ether, and examples thereof include enol ethers in which a triisopropyl silyl group or a tert-butyldimethyl silyl group is substituted.

The present invention also provides a polysubstituted cyclobutene compound that is synthesizable by the method of the present invention described above.

Another aspect of the present invention provides a method for manufacturing a compound having a bicyclo[4.2.0]octane main chain that is indicated by Chemical Formula (15): 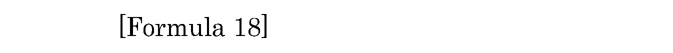 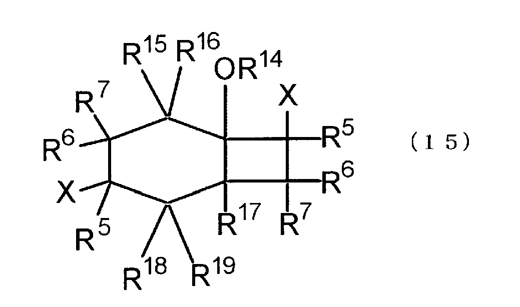 (wherein X, R⁵ through R⁷, and R¹⁴ through R¹⁹ are the same as below) by reacting an unsaturated hydrocarbon compound indicated by Chemical Formula (2) with a diene compound indicated by Chemical Formula (14): 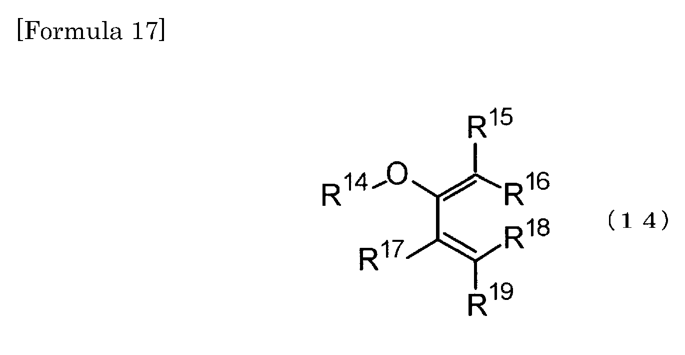 (wherein R¹⁴ is an optionally substituted alkyl group, an optionally substituted aryl group, an optionally substituted heterocyclic group, or a silyl group; R¹⁵ through R¹⁹ are each independently a hydrogen atom, a halogen atom, an optionally substituted alkyl group, an optionally substituted aryl group, an optionally substituted heterocyclic group, or an optionally substituted alkoxy group, and are the same or different; and the substituents of R¹⁴ through R¹⁹ may be bonded to each other); wherein the method for manufacturing a compound having a bicyclo[4.2.0]octane main chain is characterized in that a Brønsted acid catalyst is made to act in a non-aqueous solvent. The Brønsted acid is preferably a trifluoromethane sulfonimide, a pentafluorobenzene sulfonimide, bis(pentafluoroethyl)phosphoric acid, or a strong acid such as one having a substituted polyfluoroalkyl sulfonic acid group indicated by Chemical Formula (6). Examples of this type of acid include bis(trifluoromethane)sulfonimide, bis(pentafluoroethane)sulfonimide, bis(pentafluorobenzene)sulfonimide, N-pentafluorobenzene sulfonyl-N-trifluoromethane sulfonimide, N-trifluoromethane sulfonyl-N-trifluoromethane sulfonimide, trifluoromethane sulfonic acid, pentafluoroethane sulfonic acid, tris(trifluoromethanesulfonyl)methane, tris(pentafluorobenzenesulfonyl)methane, bis(pentafluorobenzenesulfonyl)torfluoromethane (*3) sulfonyl methane, and the like. However, these compounds are merely examples, and the present invention is not limited by the abovementioned compounds.

The present invention also provides a compound that has in the component structure thereof a bicyclo[4.2.0]octane indicated by Chemical Formula (12) that is synthesizable by the method of the present invention described above.

### [Effect of the Invention]

The present invention enables the efficient manufacture of a polysubstituted cyclobutane compound, a polysubstituted cyclobutene compound, or a polysubstituted bicyclo[4.2.0]octane compound at a high chemical yield and with excellent stereoselectivity in reaction conditions that are free of factors relating to the quantity of catalyst, the solvent, and environmental contamination by byproducts and the like. Starting materials can also be used that have high generality and are easily obtained for organic synthesis, such as an enol ether indicated by Chemical Formula (1) or a diene compound indicated by Chemical Formula (14), and an alkene compound indicated by Chemical Formula (2) or an alkyne compound indicated by Chemical Formula (11). In the present method, the amount of catalyst used can be reduced to 1/200 or less of the amount of catalyst used in the best publicly known reaction, the reaction can be performed at or near room temperature, and a polysubstituted cyclobutane compound, a polysubstituted cyclobutene compound, or a polysubstituted bicyclo[4.2.0]octane compound can be manufactured without the use of methylene chloride and other halogenated solvents that cause environmental contamination. A desired functional molecule, pharmaceutical product, or the like can also be synthesized using the compounds that are synthesizable by the present manufacturing method.

Other objects, characteristics, superior properties, and aspects of the present invention will be apparent to one skilled in the art from the description given below. However, it should be understood that the following description and the description of the present specification including specific working examples and the like merely show preferred modes of the present invention, and are given only by way of explanation. It will be clearly apparent to one skilled in the art from the information given in the following description and other portions of the present specification that various changes and/or improvements (or modifications) are possible within the intention and scope of the present invention as disclosed in the present specification. All patent references and other references cited in the present specification are cited for descriptive purposes, and the contents of the references shall be construed as being included in the disclosure of the present specification as part of the present specification.

### BEST MODE FOR CARRYING OUT THE INVENTION

Embodiments of the present invention will be described hereinafter.

The "alkyl group" in the present specification may be a straight chain or a branched chain, the "alkyl group" may be saturated or unsaturated, and the "alkyl group" may also be cyclic. Examples of the "alkyl group" include C₁₋₂₂ alkyls (examples of which are methyl, ethyl, propyl, isopropyl, butyl, isobutyl, sec-butyl, tert-butyl, pentyl, isopentyl, tert-pentyl, hexyl, heptyl, octyl, nonyl, decanyl, hexadecanyl, eicosanyl, and the like), C₂₋₂₄ alkenyls (examples of which are vinyl, allyl, isopropenyl, 1-butenyl, 2-butenyl, 3-butenyl, 2-methyl-2-propenyl, 1-methyl-2-propenyl, 2-methyl-1-propenyl, and the like), C₂₋₆ alkynyls (examples of which are ethynyl, propargyl, 1-butynyl, 2-butynyl, 3-butynyl, 1-hexynyl, and the like), C₃₋₈ cycloalkyls (examples of which are cyclopropyl, cyclobutyl, cyclopentyl, 2-cyclopentene-1-yl, cyclohexyl, 1, 3-cyclohexadienyl, cycloheptyl, spiro [4.5] decanyl, and the like), and the like. A C₁₋₆ alkyl (examples of which are methyl, ethyl, propyl, isopropyl, butyl, tert-butyl, and the like) is preferred.

Examples of the "aryl group" include C₆₋₁₄ aryls (examples of which are phenyl, 1-naphthyl, 2-naphthyl, 2-biphenylyl, 3-biphenylyl, 4-biphenylyl, 2-anthryl, 3-indenyl, 5-fluorenyl, and the like) and the like.

Examples of the "heterocyclic group" include heterocycles having 5 to 14 members that include 1 to 4 of 1 to 3 types of hetero atoms other than carbon that are selected from nitrogen, sulfur, and oxygen, and may be monocyclic, bicyclic, tricyclic, or tetracyclic. The "heterocyclic group" is preferably a monovalent group or the like that is formed by removing one hydrogen atom from any one of (i) a 5- to 14-membered (5 to 10 members are preferred, and 5 to 6 members are particularly preferred) aromatic heterocycle, (ii) a 5- to 10-membered (5 to 6 members are preferred) non-aromatic heterocycle, or (iii) a 7-to 10-membered heterocyclic bridge ring. Examples of the abovementioned 5- to 14-membered (preferably 5- to 6-membered) aromatic heterocycle include thiophene, benzo[b]thiophene, furan, benzo[b]furan, benzimidazole, oxazole, benzoxazole, isoxazole, benzisoxazole, thiazole, benzothiazole, isothiazole, benzisothiazole, oxadiazole, oxathiazole, naphtho[2,3-b]thiophene, naphtho[2,3-b]furan, pyrrole, imidazole, pyrazole, triazole, tetrazole, pyran, thyine, pyridine, pyrimidine, pyridazine, indole, isoindole, 1H-indazole, purine, pteridine, 4H-quinolidine, isoquinoline, quinoline, phthalazine, naphthylidine, quinoxaline, quinazoline, cinnoline, carbazole, β-carboline, phenanthridine, acridine, phenazine, triazine, thiazine, phenothiazine, furazan, oxazane, phenoxazine, oxadiazine, thiadiazine, oxadiazine, and other aromatic heterocycles, a ring formed by condensation with an aromatic ring (a benzene ring or the like, for example) of one or a plurality (preferably 1 or 2) of the abovementioned rings (preferably monocyclic), or a ring or the like formed by condensing heterocycles selected from the abovementioned heterocycles. Examples of the abovementioned "5- to 10-membered non-aromatic heterocycle" include pyrrolidine, imidazoline, imidazolidine, pyrazolidine, pyrazoline, piperidine, piperazine, morpholine, thiomorpholine, dioxazole, oxadiazoline, thiadiazoline, triazoline, thiadiazole, dithiazole, and the like. Examples of the abovementioned "7- to 10-membered heterocyclic bridge ring" include quinuclidine, 7-azabicyclo[2.2.1]heptane, and the like.

The "heterocyclic group" is preferably a (monocyclic or bicyclic) heterocyclic group having 5 to 14 members (preferably 5 to 10 members) that preferably includes 1 to 4 of 1 or 2 types of hetero atoms other than carbon that are selected from nitrogen, sulfur, and oxygen. Specific examples are 2-thienyl, 3-thienyl, 2-furyl, 3-furyl, 2-pyridyl, 3-pyridyl, 4-pyridyl, 2-quinonyl, 3-quinonyl, 4-quinonyl, 5-quinonyl, 8-quinonyl, 1-isoquinonyl, 3-isoquinonyl, 4-isoquinonyl, 5-isoquinonyl, pyrazinyl, 2-pyrimidinyl, 4-pyrimidinyl, 5-pyrimidinyl, 3-pyrolyl, 2-imidazolyl, 4-imidazolyl, 3-pyridazinyl, 2-thiazolyl, 4-thiazolyl, 3-isothiazolyl, 2-oxazolyl, 4-oxazolyl, 3-isooxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl, and other aromatic heterocyclic groups; and 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino, and other non-aromatic heterocyclic groups and the like. Among these examples, a 5- to 6-membered heterocyclic group or the like that includes 1 to 3 hetero atoms other than carbon that are selected from nitrogen, sulfur, and oxygen is more preferred. Specific examples thereof include 2-thienyl, 3-thienyl, 2-pyridyl, 3-pyridyl, 4-pyridyl, 2-furyl, 3-furyl, pyrazinyl, 2-pyrimidinyl, 3-pyrolyl, 3-pyridazinyl, 3-isothiazolyl, 3-isooxazolyl, 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino, 8-azabicyclo[3.2.1]octane, 9-azabicyclo[3.3.1]nonane, 3-oxa-9-azatricyclo[3.3.1.0^{2,4}]nonane, indolo[4,3,-f,g]quinoline, imidazopyrimidine, pyrazolopyrimidine, triazolo[4,3-a] [1,4]benzodiazepine, imidazopyridine, naphthylidine, pyridopyrimidine, 1-azabicyclo[3,2,0]heptane; a 4-thia- or 4-oxa-1-azabicyclo[3,2,0]heptane-ring-based heterocyclic group having a penicillin main chain, an oxapenicillin main chain, or the like; a 5-thia- or 5-oxa-1-azabicyclo[4,2,0]octane-ring-based heterocyclic group such as one having a cephalosporin main chain; a residue of streptomycin, kanamycin, dibekacin, amikacin, or another aminoglycoside-based antibiotic; a residue of erythromycin, oleandomycin, josamycin, or another macrolide-based antibiotic; a residue of vancomycin or another peptide-based antibiotic; and the like.

Examples of the "halogen atom" include fluorine, chlorine, bromine, iodine, and the like.

The "alkyl components" of the "alkyl alkoxy group" are the type of groups described above as "alkyl groups," and examples of the "alkyl alkoxy group" include C₁₋₆ alkoxy (methoxy, ethoxy, propoxy, isopropoxy, butoxy, isobutyloxy, sec-butyloxy, tert-butoxy, pentyloxy, isopentyloxy, tert-pentyloxy, hexyloxy, and the like, for example) groups having the abovementioned "alkyl groups."

The "alkyl components" of the "alkoxy group" are the type of groups described above as "alkyl groups," and examples of the "alkoxy group" include C₁₋₆ alkoxy (methoxy, ethoxy, propoxy, isopropoxy, butoxy, isobutyloxy, sec-butyloxy, tert-butoxy, pentyloxy, isopentyloxy, tert-pentyloxy, hexyloxy, and the like, for example) groups and the like.

When "substituents may be bonded to each other," the substituents may be bonded to each other to form a carbon-carbon chemical bond, or may be bonded to each other to form a ring. A ring in which the substituents are bonded to each other may be a 3- to 10-membered isocyclic or heterocyclic ring that may have at least one carbon-carbon double bond. Examples of a 3- to 10-membered isocyclic ring include cyclopropane, cyclobutane, cyclopentane, cyclohexane, cycloheptane, cyclopentene, cyclohexene, cyclopentadiene, cyclohexa-1,3-diene, cyclohexa-1,4-diene, benzene, and the like. A 3- to 10-membered heterocyclic ring may be appropriately selected from the examples cited above, for example.

Examples of the "substituent" in the "optionally substituted alkyl group," the "optionally substituted aryl group," the "optionally substituted heterocyclic group," and the "optionally substituted alkyl alkoxy group," include oxo, thioxo, an optionally substituted imino, a halogen atom (e.g., fluorine, chlorine, bromine, iodine), C₁₋₃ alkylene dioxy (e.g., methylene dioxy, ethylene dioxy), nitro, cyano, C₁₋₆ alkyl, C₂₋₆ alkenyl, carboxy C₂₋₆ alkenyl (e.g., 2-carboxyethenyl, 2-carboxy-2-methylethenyl), C₂₋₆ alkenyl, C₃₋₆ cycloalkyl, C₆₋₁₄ aryl (e.g., phenyl, 1-naphthyl, 2-naphthyl, 2-biphenylyl, 3-biphenylyl, 4-biphenylyl, 2-anthryl), C₁₋₈ alkoxy, C₁₋₆ alkoxy-carbonyl-C₁₋₆ alkoxy (e.g., ethoxycarbonyl methyloxy), hydroxy, C₆₋₁₄ aryloxy (e.g., phenyloxy, 1-naphthyloxy, 2-naphthyloxy), C₇₋₁₆ aralkyloxy (e.g., benzyloxy, phenethyloxy), mercapto, C₁₋₆ alkylthio, C₆₋₁₄ arylthio (e.g., phenylthio, 1-naphthylthio, 2-naphthylthio), C₇₋₁₆ aralkylthio (e.g., benzylthio, phenethylthio), amino, mono-C₁₋₆ alkylamino (e.g., methylamino, ethylamino), mono-C₆₋₁₄ arylamino (e.g., phenylamino, 1-naphthylamino, 2-naphthylamino), di-C₁₋₆ alkylamino (e.g., dimethylamino, diethylamino, ethylmethylamino), di-C6-₁₄ arylamino (e.g., diphenylamino), formyl, carboxy, C₁₋₆ alkyl-carbonyl (e.g., acetyl, propionyl), C₃₋₆ cycloalkyl-carbonyl (e.g., cyclopropyl carbonyl, cyclopentyl carbonyl, cyclohexyl carbonyl), C₁₋₆ alkoxy-carbonyl (e.g., methoxycarbonyl, ethoxycarbonyl, propoxycarbonyl, tert-butoxycarbonyl), C₆₋₁₄ aryl-carbonyl (e.g., benzoyl, 1-naphthoyl, 2-naphthoyl) C₇₋₁₆ aralkyl-carbonyl (e.g., phenylacetyl, 3-phenylpropionyl), C₆₋₁₄ aryloxy-carbonyl (e.g., phenoxycarbonyl), C₇₋₁₆ aralkyloxy-carbonyl (e.g., benzyloxy carbonyl, phenethyloxy carbonyl), 5- or 6-membered heterocyclic carbonyl (e.g., nicotinoyl, isonicotinoyl, thenoyl, furoyl, morpholino carbonyl, thiomorpholino carbonyl, piperadine-1-ylcarbonyl, pyrrolidine-1-ylcarbonyl), carbamoyl, thiocarbamoyl, mono-C₁₋₆ alkyl-carbamoyl (e.g., methylcarbamoyl, ethylcarbamoyl), di-C₁₋₆ alkyl-carbamoyl (e.g., dimethylcarbamoyl, diethylcarbamoyl, ethyl methyl carbamoyl), C₆-₁₄ aryl-carbamoyl (e.g., phenylcarbamoyl, 1-naphthylcarbamoyl, 2-naphthylcarbamoyl), 5- or 6-membered heterocyclic carbamoyl (e.g., 2-pyridylcarbamoyl, 3-pyridylcarbamoyl, 4-pyridylcarbamoyl, 2-thienylcarbamoyl, 3-thenylcarbamoyl), C₁₋₆ alkylsulfonyl (e.g., methylsulfonyl, ethylsulfonyl), C₆₋₁₄ arylsulfonyl (e.g., phenylsulfonyl, 1-naphthylsulfonyl, 2-naphthylsulfonyl), C₁₋₆ alkylsulfinyl (e.g., methylsulfinyl, ethylsulfinyl), C₆₋₁₄ arylsulfinyl (e.g., phenylsulfinyl, 1-naphthylsulfinyl, 2-naphthylsulfinyl), formylamino, C₁₋₆ alkyl-carbonylamino (e.g., acetylamino), C₆₋₁₄ arylcarbonylamino (e.g., benzoylamino, naphthylamino), C₁₋₆ alkoxy-carbonylamino (methoxycarbonyl amino, ethoxycarbonyl amino, propoxycarbonyl amino, butoxycarbonyl amino), C₁₋₆ alkylsulfonyl amino (e.g., methylsulfonyl amino, ethylsulfonyl amino), C₆₋₁₄ arylsulfonyl amino (e.g., phenylsulfonyl amino, 2-naphthylsulfonyl amino, 1-naphthylsulfonyl amino), C₁₋₆ alkyl-carbonyloxy (e.g., acetoxy, propionyloxy), C₆₋₁₄ aryl-carbonyloxy (e.g., benzoyloxy, naphthylcarbonyloxy), C₁₋₆ alkoxy-carbonyloxy (e.g., methoxycarbonyl oxy, ethoxycarbonyl oxy, propoxycarbonyl oxy, butoxycarbonyl oxy), mono-C₁₋₆ alkyl-carbamoyloxy (e.g., methylcarbamoyl oxy, ethylcarbamoyl oxy), di-C₁₋₆ alkyl-carbamoyloxy (e.g., dimethylcarbamoyl oxy, diethylcarbamoyl oxy), C₆₋₁₄ aryl-carbamoyl oxy (e.g., phenylcarbamoyl oxy, naphthylcarbamoyloxy), nicotinoyloxy, a 5- to 7-membered saturated optionally substituted cyclic amino, a 5- to 10-membered aromatic heterocyclic group (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl), sulfo, sulfamoyl, sulfinamoyl, sulfenamoyl, and the like. The "alkyl component" (including the alkyl component in an alkoxy), "alkylene component," "alkenyl component," "alkynyl component," "aryl component," and "heterocyclic component" in the substituents cited herein may be substituted with any one or more substituents, in which case the substituent may be of a type described above. The substituent in "an optionally substituted compound" as described above may also be of a type described above.

An optionally substituted trialkylsilyl, triarylsilyl, or dialkylmonoarylsilyl, or a -SiR⁸R⁹R¹⁰ or the like, for example, may be used as the "silyl group." The substituent used is a halogen atom (e.g., fluorine, chlorine, bromine, iodine), a formyl, a C₁₋₆ alkyl-carbonyl (e.g., acetyl, propionyl, valeryl), a nitro group, or the like, and the number of substituents is about 1 to 3. Examples of typical silyl groups include trimethylsilyl, triethylsilyl, dimethylethylsilyl, diethylmethylsilyl, tert-butyldimethylsilyl, tert-butyldiethylsilyl, triisopropylsilyl, isopropyldimethylsilyl, isopropyldiethylsilyl, triphenylsilyl, dimethylphenylsilyl, and the like.

The term "Brønsted acid" refers to a proton donor, and a Brønsted acid that is suitable for the reaction of the present invention is known as a super Brønsted acid. The Brønsted acid may also be a polymer-supported super Brønsted acid. This type of Brønsted acid is described above, and an example thereof is a polyfluoro-substituted sulfonyl compound indicated by Chemical Formula (6). More specifically, the bis(polyfluoroalkylsulfonyl)imide indicated by Chemical Formula (7), the bis(polyfluoroalkylsulfonyl)methane indicated by Chemical Formula (8), and the tris(polyfluoroalkylsulfonyl)methide indicated by Chemical Formula (9) can be cited, and examples thereof include 1-[bis(trifluoromethanesulfonyl)methyl]-2,3,4,5,6-penta-fluorobenzene, 1-[bis(trifluoromethanesulfonyl)methyl]-4-(1H,1H-perfluorotetradecyloxy)-2,3,5,6-tetrafluorobenzene, bis(trifluoromethanesulfonyl)methyl-tetrafluorophenyl polystyrene resin, and the like. Except for the substitution of the hydrogen atom with two or more fluorine atoms, the "alkyl group," "aryl group," and "heterocyclic group" in the "heterocyclic group, aryl group, or alkyl group in which two or more fluorine atoms are substituted" may be the same as the groups described above. A type of group in which half or more of the hydrogen atoms present in the substituent are substituted with fluorine atoms is suitable, and it is permissible and sometimes preferable for all of the hydrogen atoms present in the substituent to be substituted with fluorine atoms. Typical examples of this type of group are trifluoromethyl, pentafluoroethyl, heptafluoropropyl, nonafluorobutyl, undecafluoropentyl, tridecafluorohexyl, pentadecafluoroheptyl, heptadecafluorooctyl, pentafluorophenyl, p-trifluoromethyl tetrafluorophenyl, and the like.

The "nitrogen atom, oxygen atom, carbon atom, sulfur atom, or phosphorus atom that has a plurality of substituents or is unsubstituted as chemically allowed" includes residues derived from N-R, O, C(R)₂, CHR, CH₂, S, and phosphoric acid.

The "organic group" may be an optionally substituted alkyl group, an optionally substituted aryl group, or an optionally substituted heterocyclic group, or a residue of polystyrene or another polymer.

For example, a polymer-supported super Brønsted acid may be used that swells well in polar non-aqueous solvents and non-polar non-aqueous solvents. This polymer-supported super Brønsted acid is convenient as a recoverable, reusable solid acid catalyst, can be quantitatively recovered by filtration after the reaction, and can be reused ten or more times without losing catalyst activity, and is thus superior. The polymer-supported super Brønsted acid also has application as a filler in a reaction column. For example an extremely simple structure formed merely by filling a disposable syringe with a mixture of cerite and the polymer-supported super Brønsted acid may be used as the reaction column, and the reaction column may be connected to a pump to construct a continuous flow system. When the catalyst used has an extremely high level of catalyst activity, an advantage is gained in that a small sample can be converted at a high yield in a single flow in a short time. A super Brønsted acid that has enhanced fluorous properties may be dissolved in cyclohexane or another solvent while heated under reflux in the reaction, and made to function as the catalyst. After the reaction, the Brønsted acid may be precipitated upon returning to room temperature, and recovered/reused. In such a case, the super Brønsted acid is useful and convenient. It is possible to enable a higher activity to be obtained than from a solid catalyst using the super Brønsted acid as a uniform catalyst in this manner, and advantages can be gained in that the catalyst can be recovered as a solid, and it is possible to eliminate the need for a fluorous solvent for recovering/reusing the catalyst.

It is advantageous to perform the reaction of the present invention without a solvent (which may include cases in which a reaction starting material also functions as a solvent) or in the presence of a solvent that is inactive in the reaction. The solvent is not particularly limited insofar as the solvent is non-aqueous and the reaction is allowed to progress, and examples of solvents include methanol, ethanol, n-propanol, isopropanol, cyclohexanol, furfuryl alcohol, ethylene glycol, benzyl alcohol, and other alcohols; benzene, toluene, xylene, ethyl benzene, cumene, and other aromatic hydrocarbons; solvent naphtha, petroleum ether, ligroin, dichloromethane, dichloroethane, chloroform, dichlorohexane, and other halogenated hydrocarbons; 2-methylbutane, n-hexane, isohexane, n-heptane, octane, nonane, decane, 2-methylpentane, 2,2-dimethylbutane, 2,3-dimethylbutane, 2,2,3-trimethylheptane, cyclopentane, methylcyclopentane, cyclohexane, methylcyclohexane, decahydronaphthalene, and other saturated hydrocarbons or aliphatic hydrocarbons; diethyl ether, isopropyl ether, isoamyl ether, tetrahydrofuran (THF), dioxane, tetrahydrofurfuryl alcohol, diethylene glycol, cyclohexylmethyl ether, methyl Cellosolve, Cellosolve, butyl Cellosolve, methyl tert-butanol, and other ethers; acetone, methyl ethyl ketone, furfural, methyl isobutyl ketone, mesityl oxide, diacetone alcohol, cyclohexanone, and other ketones; acetonitrile, benzonitrile, and other nitriles; dimethyl sulfoxide (DMSO), sulfolane, and other sulfoxides; formamide, N,N-dimethyl formamide (DMF), N,N-dimethyl acetamide, and other amides; methyl formate, ethyl formate, ethyl acetate, butyl acetate, methoxybutyl acetate, cellosolve acetate, diethyl carbonate, glycol carbonates, and other esters; formic acid, acetic acid, propionic acid, acetic anhydride, and other organic acids; hexamethyl phosphorotriamide, pyridine, quinoline, and other heterocyclic compounds; aniline, N-methylaniline, and other aromatic amines; and nitro compounds and the like. These solvents may be used singly, or a mixture in which two or more types of these solvents are mixed in an appropriate ratio, e.g., 1:1 to 1:10, may be used as needed.

The amount of the Brønsted acid catalyst used in the reaction of the present invention may be a catalytic amount with respect to the enol ether compound or diene compound, or with respect to the alkene compound in which a carbonyl group is substituted at the 1-position or alkyne compound in which a carbonyl group is substituted at the 1-position. The term "catalytic amount" refers to a quantity that is small in relation to the quantities of the compounds. For example, the quantity of the catalyst may be 1/1.1 to 1/10,000 of the quantity of the compounds, and is usually 1/5 to 1/5,000 of the quantity of the compounds. The catalyst quantity in some cases is 1/10 to 1/2,000, 1/20 to 1/1,000, 1/50 to 1/500, or another fraction of the quantity of the compounds, but it is apparent that an appropriate quantity may be freely selected according to the combination of starting material compounds.

The reaction temperature of the present reaction is usually -100°C to 200°C, preferably -80°C to 150°C, more preferably -10°C to 100°C, and more preferably 0°C to 50°C. A temperature of 0°C to room temperature is suitable as the reaction temperature, and the reaction time is usually in the range of 1 minute to 2 weeks, preferably 5 minutes to 50 hours, more preferably 10 minutes to 35 hours, and more preferably 15 minutes to 20 hours. The product may be used in the subsequent reaction in crude form or as the unmodified reaction liquid, but the product may be isolated from the reaction mixture according to the usual method, and can be isolated and purified by condensation, vacuum condensation, distillation, fractionation, solvent extraction, liquid conversion, and solvent exchange; high-performance liquid chromatography (HPLC), thin layer chromatography (TLC), column chromatography, and other chromatography methods, for example; and crystallization, re-crystallization, and other methods.

The compound having a bicyclo[4.2.0]octane main chain that is a compound selected from the group consisting of a compound indicated by Chemical Formula (3), a compound indicated by Chemical Formula (12), and a compound indicated by Chemical Formula (15) may be in the form of a salt or in an isolated state. Examples of the salt include metal salts, ammonium salts, salts of organic bases, salts of inorganic acids, salts of organic acids, salts of basic or acidic amino acids, and the like. Examples of preferred metal salts include salts of sodium, potassium, and other alkali metals; salts of calcium, magnesium, barium, and other alkaline earth metals; and aluminum salts and the like. Examples of preferred salts of organic bases include salts of trimethyl amines, triethylamines, pyridines, picolines, 2,6-lutidines, ethanol amines, diethanol amines, triethanol amines, cyclohexyl amines, dicyclohexyl amines, N,N'-dibenzylethylene diamines, and the like. Examples of preferred salts of inorganic acids include salts of hydrochloric acid, hydrobromic acid, nitric acid, sulfuric acid, phosphoric acid, and the like. Examples of preferred salts of basic amino acids include salts of arginines, lysines, ornithines, and the like. Examples of preferred salts of acidic amino acids include salts of asparagines, glutamines, and the like. Among these examples, non-toxic salts are preferred. For example, when there is an acidic functional group in the compound, it is possible to use an alkali metal salt (e.g., a sodium salt, a potassium salt, or the like), an alkaline earth metal salt (e.g., a calcium salt, a magnesium salt, a barium salt, or the like), or other inorganic salt, an ammonium salt, or the like; and when there is a basic functional group in the compound, it is possible to use a salt of hydrochloric acid, hydrobromic acid, nitric acid, sulfuric acid, phosphoric acid, or another inorganic acid; or a salt of acetic acid, phthalic acid, fumaric acid, oxalic acid, tartaric acid, maleic acid, citric acid, succinic acid, methane sulfonic acid, p-toluene sulfonic acid, or another organic acid.

The present invention provides a method for manufacturing a polysubstituted cyclobutane compound that has low environmental load (ecologically advantageous) and high stereoselectivity that is applicable to industrial manufacturing from the standpoint of operation, substrate generality, catalyst, solvent, and efficiency. The desired substance can be synthesized with a high chemical yield by the method of the present invention, and the obtained product can be advantageously utilized as an intermediate for synthesizing functional molecules, pharmaceuticals, agricultural chemicals, and other organic compounds and the like.

The present invention will be described hereinafter using specific working examples, but the working examples are provided merely for reference to specific embodiments in order to describe the present invention. These examples are given to describe specific embodiments of the present invention, but do not limit or restrict the scope of the invention disclosed in the present application. It is apparent that various embodiments of the present invention are possible based on the idea of the present specification.

All of the working examples have been or can be implemented using standard techniques unless otherwise specified, and these techniques are known to and commonly used by those skilled in the art.

### [Working Example 1]

Manufacture of polysubstituted cyclobutane compound 3b

In an argon atmosphere, a methylene chloride solvent (10 mL) of 1-tert-butyldimethylsiloxy-2-methyl-1-cyclohexene (1b: 622 mg, 2.75 mmol) and methyl acrylate (2a: 225 µL, 2.5 mmol) was cooled to -78°C, bis(trifluoromethane)sulfonimide (0.08 M toluene solution, 31 µL, 2.5 µmol) was dripped into the solution, and the solution was stirred for two hours at the same temperature. A saturated bicarbonate solution was added to the reaction solution, and extraction was then performed using hexane. After the organic layer was dried using magnesium sulfate, the product was condensed using an evaporator. The residue was purified by silica gel chromatography (hexane:diethyl ether = 50:1), and (1R*, 6S*, 8R*)-1-(tert-butyldimethylsiloxy)-8-methoxycarbonyl-6-methylbicyclo[4.2.0]octane (compound trans-3b) was obtained.

Yield: 766 mg, yield ratio: 98%, diastereoselectivity: 100%. Table 1 also shows the results obtained when bis(trifluoromethane)sulfonimide was used in a ratio of 1 mol% with respect to the starting material.
Colorless oily substance; IR (neat) 2960, 2858, 1732, 1464, 1292, 1223, 1178, 1097, 837, 775 cm⁻¹; ¹H NMR (400 MHz, CDCl₃) · 3.68 (s, 3H), 3.16 (t, 1H, J = 8.8 Hz), 1.83 (t, 1H, J = 10.4 Hz), 1.67 (m, 2H), 1.57-1.14 (m, 7H), 1.08 (s, 3H), 0.89 (s, 9H), 0.15 (s, 3H), 0.10 (s, 3H); ¹³C NMR (75 MHz, CDCl₃) · 173.7, 50.9, 48.3, 41.1, 33.1, 32.0, 26.6, 25.9, 25.7, 24.6, 21.4, 20.0, 18.3, -3.2, -3.5; LRMS (EI) m/z 255 (M-57)⁺. Anal calcd for C₁₇H₃₂O₃Si: C, 65.33; H, 10.32, found C, 65.38; H, 10.11.

As is apparent from these results, when bis(trifluoromethane)sulfonimide was used in the ratio of 0.1 mol% (substrate:catalyst = 1000:1) with respect to the starting material, the polysubstituted cyclobutane compound was obtained in a quantitative yield ratio of 98%, and the diastereomer selectivity in this case was 100:0, with trans isomers emphasized in relation to cis isomers.

### [Comparative Example 1]

Yield ratio of polysubstituted cyclobutane compound 3b when EtAlCl₂ was used as the catalyst

An experiment was conducted under the same conditions as Working Example 1 in order to compare the performance of catalysts using ethyl aluminum dichloride instead of bis(trifluoromethane)sulfonimide as the catalyst.

The yield ratio of compound trans-3b was 23% when the catalyst was used in the ratio of 2 mol% (substrate:catalyst = 50:1) Compound trans-3b could not be obtained when 0.1 mol% (substrate:catalyst = 1000:1) of the catalyst was used.

### [Working Example 2]

Table 1 below shows the product, yield ratio, and diastereomer ratio when 1 mol% of bis(trifluoromethane)sulfonimide was reacted with enol ethers 1a through 1f and alkene compounds 2a through 2c in a methylene chloride solvent.

In Table 1, TBS = tert-butyldimethylsilyl group, Me = methyl group, Ph = phenyl group, and ⁱPr = isopropyl group.

**[Table 1]**

| Yield ratio and stereoselectivity of a polysubstituted cyclobutane compound | | | | | | | | |
|---|---|---|---|---|---|---|---|---|
| Starting materials | | | | Products (cyclobutane) | | | Total yield ratio | Diastereomer ratio (trans : cis) |
| Enol ethers | | Alkene | | Trans isomers | | Cis isomers | | |
| 1a | 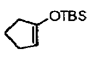 | 2a | 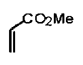 | 3a | 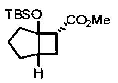 | 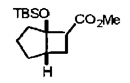 | 77 | 100 : 0 |
| 1b | 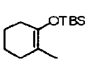 | 2a | 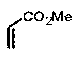 | 3b | 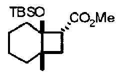 | 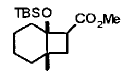 | 92 | 100 : 0 |
| 1c | 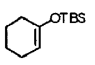 | 2a | 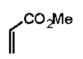 | 3c | 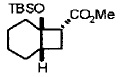 | 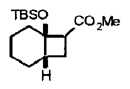 | 70 | 80 : 20 |
| 1d | 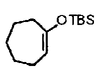 | 2a | 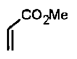 | 3d | 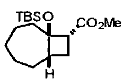 | 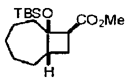 | 91 | 93 : 7 |
| 1e | 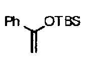 | 2a | 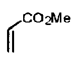 | 3e | 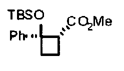 | 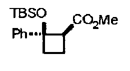 | 93 | 81 : 19 |
| 1f | 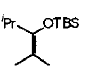 | 2a | 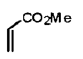 | 3f | 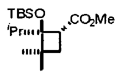 | 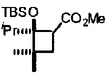 | 75 | 72 : 28 |
| 1d | 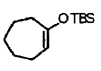 | 2b | 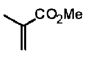 | 3g | 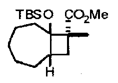 | 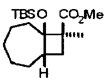 | 71 | 87: 13 |
| 1d | 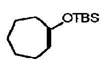 | 2c | 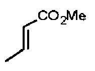 | 3h | 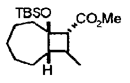 | 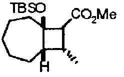 | 75 | 67 : 33 |

The spectral data of the compounds obtained in the abovementioned working examples are shown below.

### trans-3a

Colorless oily substance; IR (neat) 1738 cm⁻¹; ¹H NMR (600MHz, CDCl₃) δ 3.15 (s, 3H) 2.94 (t, J = 7.8 Hz, 1H), 2.57 (m, 2H), 1.86 (m, 2H), 1.75 (m, 2H), 1.57 (m, 1H), 1.50 (d, J = 6.6 Hz, 1H), 1.22 (m, 1H), 0.83 (s, 9H), 0.07 (s, 3H), 0.02 (s, 3H); ¹³C NMR (150 MHz, CDCl₃) δ 172.8, 85.6, 51.3, 48.3, 43.4, 41.5, 31.6, 25.6 (3C), 23.8, 20.4, 17.8, -2.9, -3.2; LRMS m/z 227 (M⁺-57); HRMS calcd for C₁₁H₁₉O₃S₁: 227.1103, found: 227.1103.

### trans-3d

Colorless oily substance; IR (neat) 2928, 1738, 1462, 1435, 1360, 1337, 1258, 1198, 1132, 1007, 901, 837 cm⁻¹; ¹H NMR (600MHz, CDCl₃) δ 3.68 (s, 3H) 3.09 (t, 1H, J = 9.6 Hz), 2.31 (m, 1H), 2.23 (m, 1H), 1.94 (m, 1H), 1.83-1.62 (m, 4H), 1.58-1.49 (m, 3H), 1.27-1.14 (m, 3H), 0.90 (s, 9H), 0.19 (s, 3H), 0.13 (s, 3H); ¹³C NMR (75 MHz, CDCl₃) δ 172.9, 82.1, 51.0, 48.3, 47.1, 34.5, 32.9, 32.0, 26.5, 25.7 (3C), 23.5, 21.8, 18.0, -2.9, -3.1; LRMS m/z 225 (M-57)⁺.

### trans-3e

Colorless oily substance; IR (neat) 2953, 2856, 1737, 1435, 1344, 1250, 1198, 1175, 1142, 1003, 837, 777, 700 cm⁻¹; ¹H NMR (300MHz, CDCl₃) δ 7.62-7.54 (m, 2H) 7.42-7.24 (m, 3H), 3.32 (m, 1H), 2.80 (1H), 2.57-2.35 (m, 2H), 1.80 (m, 1H), 0.82 (s, 9H), -0.07 (s, 3H), -0.49 (s, 3H); ¹³C NMR (75 MHz, CDCl₃) δ 172.5, 145.6, 128.2 (2C), 127.6, 126.3 (2C),79.3, 53.1, 51.5, 32.2, 25.4 (3C), 17.8, 15.9, -3.3, -3.9; LRMS m/z 263 (M-57)⁺.

### cis-3e

Colorless oily substance; IR (neat) 2953, 2856, 1738, 1462, 1435, 1360, 1256, 1200, 1078, 1005, 835, 777, 700 cm⁻¹; ¹H NMR (600MHz, CDCl₃) δ 7.14 (m, 2H) 7.31-7.19 (m, 3H), 3.48 (t, 1H, J = 9.0 Hz), 3.29 (s, 3H), 2.77 (m, 1H), 2.34 (m, 1H), 2.08-1.96 (m, 2H), 0.90 (s, 9H), 0.02 (s, 3H), -0.23 (s, 3H); LRMS m/z 263 (M-57)⁺.

### trans-3f

Colorless oily substance; IR (neat) 1738 cm⁻¹; ¹H NMR (300MHz, CDCl₃) δ 3.67 (s, 3H) 3.15 (dd, J = 9.6, 6.3 Hz, 1H), 2.18 (7, J = 6.6 Hz, 1H), 1.79 (m, 2H), 1.25 (m, 3H), 1.06 (s, 3H), 0.93 (s, 9H), 0.84 (d, J = 6.6 Hz, 3H), 0.75 (d, J = 6.6 Hz, 3H), 0.24 (s, 3H), 0.20 (s, 3H); ¹³C NMR (150 MHz, CDCl₃) δ 174.7, 86.7, 51.4, 47.1, 43.2, 32.1, 31.3, 26.2 (3C), 25.7, 25.3, 19.1, 17.6, 17.3, -2.1, -2.2; LRMS m/z 257 (M⁺-57); HRMS calcd for C₁₄H₂₅O₃Si: 257.1573, found: 257.1573.

### trans-3g

Colorless oily substance; IR (neat) 1732 cm⁻¹; ¹H NMR (600MHz, CDCl₃) δ 3.67 (s, 3H) 2.45 (m, 1H), 2.00 (t, J = 10.8 Hz, 1H), 1.82-1.36 (m, 11H), 1.35 (s, 3H), 0.91 (s, 9H), 0.17 (s, 3H), 0.14 (s, 3H); ¹³C NMR (150 MHz, CDCl₃) δ 176.4, 83.2, 51.2, 50.8, 43.9, 35.2, 31.3, 30.5, 28.4, 26.1 (3C), 24.8, 23.1, 21.7, 18.7, -1.7, -1.9; LRMS m/z 269 (M⁺-57); HRMS calcd for C₁₄H₂₅O₃Si: 269.1573, found: 269.1573.

### trans-3h

Colorless oily substance; IR (neat) 1738 cm⁻¹; ¹H NMR (600MHz, CDCl₃) δ 3.65 (s, 3H) 2.65 (d, J = 9.0 Hz, 1H), 1.99 (m, 1H), 1.83 (m, 1H), 1.80-1.61 (m, 7H), 1.24-1.07 (m, 3H), 1.06 (d, J = 6.6 Hz, 3H), 0.88 (s, 9H), 0.16 (s, 3H), 0.10 (s, 3H); ¹³C NMR (150 MHz, CDCl₃) δ 172.6, 79.4, 56.6, 54.7, 51.1, · 33.5, 33.1, 32.1, 31.1, 26.2, 25.9 (3C), 23.6, 19.8, 18.2, -2.6, -2.8.

### cis-3h

Colorless oily substance; IR (neat) 1738 cm⁻¹; ¹H NMR (600MHz, CDCl₃) δ 3.62 (s, 3H) 2.85 (m, 2H), 2.55 (d, J = 10.2 Hz, 1H), 2.18 (m, 1H), 2.05 (dd, J = 14.4, 7.8 Hz, 1H), 1.94 (brm, 1H), 1.82 (brm, 1H), 1.65-1.54 (m, 3H), 1.42-1.15 (m, 4H), 0.92 (d, J = 7.2 Hz, 3H), 0.81 (s, 9H), 0.15 (s, 3H), 0.10 (s, 3H); ¹³C NMR (150 MHz, CDCl₃) d 172.0, 82.8,56.6, 51.1, 48.9, 41.8, 31.6, 29.8, 27.6, 27.3, 25.7 (3C), 24.3, 18.2, 14.3, -1.9, -2.5.

As is apparent from Table 1, the polysubstituted cyclobutane compound was obtained at a satisfactory yield ratio of approximately 70% by the present manufacturing method even when a cyclic or non-cyclic enol ether compound and a substituted or unsubstituted acrylic acid ester were used as starting materials.

### [Working Example 3]

In an argon atmosphere, an ethyl acetate solution (25 mL) of 1-tert-butyldimethylsiloxy-1-cycloheptene (1d: 5.75 g, 25.4 mmol) and methyl acrylate (2a: 2.18 mL, 24.2 mmol) was cooled to 0°C, bis(trifluoromethane)sulfonimide (0.08 M toluene solution, 1.5 mL, 120 µmol) was dripped into the solution, and the solution was stirred for 30 minutes from the temperature of 0°C to room temperature. A saturated bicarbonate solution was added to the reaction solution, and extraction was then performed using hexane. After the organic layer was dried using magnesium sulfate, the product was condensed using an evaporator. The residue was purified by silica gel chromatography (hexane:diethyl ether = 50:1), and (1R*, 7S*, 9R*)-1-(tert-butyldimethylsiloxy)-9-methoxycarbonyl-bicyclo[5.2.0]nonane (compound trans-3d) was obtained.
Yield: 7.5 g, yield ratio: 94%, diastereoselectivity: 97:3 (trans:cis).

As is apparent from the working example described above, the cyclobutane compound was obtained at a satisfactory yield ratio by the present invention even when ethyl acetate or another non-halogenated solvent was used, there was no need for cooling to an extremely low reaction temperature, and the reaction could be performed at a more easily controllable temperature range of 0°C to room temperature.

### [Working Example 4]

Table 2 below shows the products and yield ratios when 2 mol% of bis(trifluoromethane)sulfonimide was reacted with enol ethers 1a through 1d, enol ether 1f, and alkyne compound 4 in a methylene chloride solvent. In Table 2, TBS = tert-butyldimethylsilyl group, Et = ethyl group, and ⁱPr = isopropyl group.

**[Table 2]**

| Yield ratio of a polysubstituted cyclobutene compound | | | | | | |
|---|---|---|---|---|---|---|
| Starting materials | | | | | | Yield ratio |
| Enol ethers | | Alkyne | | Products (cyclobutene) | | |
| 1a | 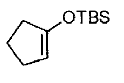 | 4 | 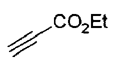 | 5a | 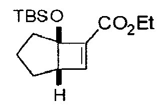 | 66 |
| 1b | 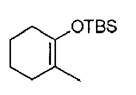 | 4 | 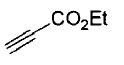 | 5b | 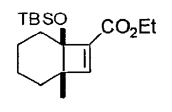 | 53 |
| 1c | 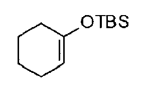 | 4 | 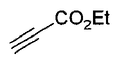 | 5c | 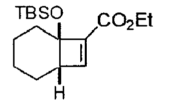 | 45 |
| 1d | 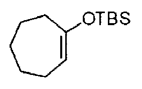 | 4 | 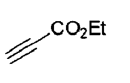 | 5d | 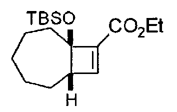 | 80 |
| 1f | 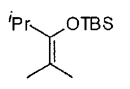 | 4 | 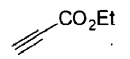 | 5f | 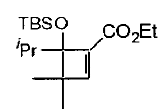 | 50 |

Manufacture of polysubstituted cyclobutene compound 5d

In an argon atmosphere, a bis(trifluoromethane)sulfonimide solution (0.08 M toluene solution, 100 µL, 8 µmol) was dripped at room temperature into a methylene chloride solution (2 mL) of 1-tert-butyldimethylsiloxy-1-cycloheptene (1d: 100 mg, 0.44 mmol) and ethyl propiolate (4:41 µL, 0.4 mmol), and the solution was stirred for two hours at the same temperature. A saturated bicarbonate solution was added to the reaction solution, and extraction was then performed using hexane. After the organic layer was dried using magnesium sulfate, the product was condensed using an evaporator. The residue was purified by silica gel chromatography (hexane:diethyl ether = 50:1), and the desired colorless oily product 5d was obtained.
Yield: 104.1 mg, yield ratio: 80%.
Colorless oily substance; IR (neat) 2928, 2855, 1715, 1607, 1472, 1319, 1250, 1198, 1113, 1070, 988, 920, 860, 837 cm⁻¹; ¹H NMR (400MHz, CDCl₃) δ 6.88 (s, 1H) 4.23-4.11 (m, 2H), 2.74 (m, 1H), 2.13 (m, 1H), 1.82 (m, 1H), 1.77-1.57 (m, 4H), 1.54-1.46 (m, 1H), 1.35-1.22 (m, 3H), 1.27 (t, 3H, J = 6.6 Hz), 0.86 (s, 9H), 0.03 (s, 3H), 0.00 (s, 3H).

The spectral data of the obtained compounds are shown in Table 2.
5b
Colorless oily substance; IR (neat) 1720 cm⁻¹; ¹H NMR (300MHz, CDCl₃) δ 6.93 (s, 1H) 4.20 (m, 2H), 2.04 (m, 1H), 1.78 (m, 1H), 1.64-1.24 (m, 6H), 1.30 (t, J = 6.9 Hz, 3H), 1.08 (s, 3H), 0.86 (s, 9H), 0.13 (s, 3H), 0.02 (s, 3H); ¹³C NMR (75 MHz, CDCl₃) δ 162.5, 154.8, 141.5, 80.1, 59.9, 51.0, 31.1, 30.3, 25.8 (3C), 21.2, 18.3, 16.9, 16.8, 14.2, -2.9, -3.2; LRMS m/z 267 (M⁺-57); Anal. Calcd for C₁₈H₃₂O₃Si: C, 66.62; H, 9.94. found: C, 66.56; H, 9.66.

As is apparent from Table 2, the polysubstituted cyclobutene compound was obtained at a yield ratio of approximately 50% or higher even when a cyclic or non-cyclic enol ether compound and a alkyne compound were used as starting materials in the present manufacturing method that uses bis(trifluoromethane)sulfonimide.

### [Working Example 5]

Manufacture of polysubstituted cyclobutene compound through the use of bis(trifluoromethane) sulfonimide

An experiment was conducted using bis(trifluoromethane sulfonyl)methane instead of bis(trifluoromethane)sulfonimide as the catalyst.

In an argon atmosphere, bis(trifluoromethane sulfonyl)methane tetrafluorophenyl-polystyrene resin (6 mg) was dripped at room temperature into a methylene chloride solution (2 mL) of 1-tert-butyldimethylsiloxy-1-cyclohexene (1b: 115 µL, 0.44 mmol) and ethyl propiolate (4:41 µL, 0.4 mmol), and the solution was stirred for one hour at the same temperature. A saturated bicarbonate solution was added to the reaction solution, and extraction was then performed using hexane. After the organic layer was dried using magnesium sulfate, the product was condensed using an evaporator. The residue was purified by silica gel chromatography (hexane:diethyl ether = 50:1), and the desired colorless oily product 5b was obtained.
The yield was 68.1 g, the yield ratio was 53%, and the results obtained were the same as when bis(trifluoromethane)sulfonimide was used.
Colorless oily substance; IR (neat) 2928, 2855, 1715, 1607, 1472, 1319, 1250, 1198, 1113, 1070, 988, 920, 860, 837 cm⁻¹; ¹H NMR (400MHz, CDCl₃) δ 6.88 (s, 1H) 4.23-4.11 (m, 2H), 2.74 (m, 1H), 2.13 (m, 1H), 1.82 (m, 1H), 1.77-1.57 (m, 4H), 1.54-1.46 (m, 1H), 1.35-1.22 (m, 3H), 1.27 (t, 3H, J = 6.6 Hz), 0.86 (s, 9H), 0.03 (s, 3H), 0.00 (s, 3H).

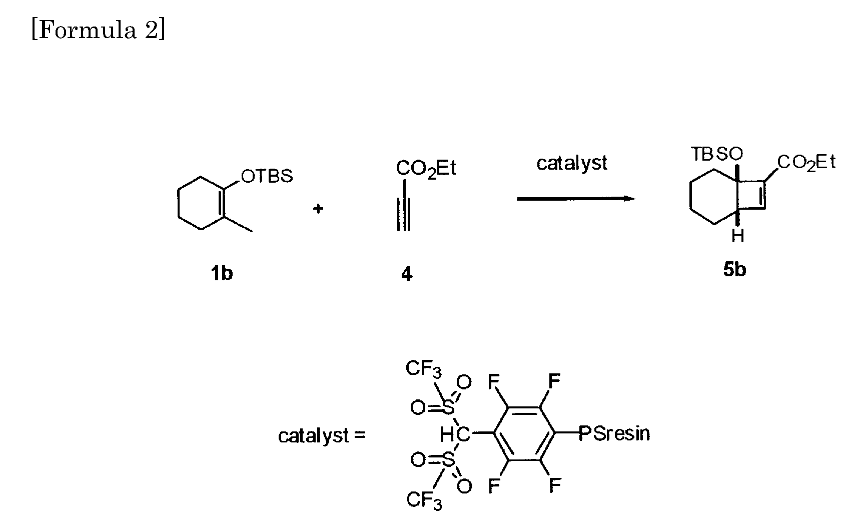

### [Working Example 6]

In an argon atmosphere, bis(trifluoromethane)sulfonimide (0.08 M toluene solution, 1.5 mL, 120 µmol) was dripped into a mixture of 1-tert-butyldimethylsiloxy-1-cycloheptene (1d: 5.75 g, 25.4 mmol) and ethyl propiolate (4:2.54 mL, 24.2 mmol), and the solution was stirred for 10 minutes at room temperature. A saturated bicarbonate solution was added to the reaction solution, and extraction was then performed using hexane. After the organic layer was dried using magnesium sulfate, the product was condensed using an evaporator. The residue was purified by silica gel chromatography (hexane:diethyl ether = 50:1), and (1R*, 7S*)-1-(tert-butyldimethylsiloxy)-9-ethoxycarbonyl bicyclo[5.2.0]nonane (compound 5d) was obtained.
Yield: 7.72g, yield ratio: 94%.

It was apparent from the working example described above that the cyclobutene compound is obtained at a satisfactory yield ratio by the present invention, which uses bis(trifluoromethane)sulfonimide, as well as under conditions in which no solvent is used.

### [Working Example 7]

Manufacture of (1S*, 4S*, 6S*, 8R*)-1-(tert-butyldimethylsiloxy)-4,8-bis(methoxycarbonyl)-6-methyl bicyclo[4.2.0]octane (7)

In an argon atmosphere, bis(trifluoromethane)sulfonimide (0.08 M toluene solution, 50 µL, 4 µmol) was dripped at room temperature into a methylene chloride solution (2 mL) of 1-tert-butyldimethylsiloxy-3-methyl-2,3-butene (6: 522 mg, 0.263 mmol) and methyl acrylate (2a: 90 µL, 1.0 mmol), and the solution was stirred for four hours at the same temperature. A saturated bicarbonate solution was added to the reaction solution, and extraction was then performed using hexane. After the organic layer was dried using magnesium sulfate, the product was condensed using an evaporator. The residue was purified by silica gel chromatography (hexane:diethyl ether = 10:1), and the desired colorless oily product 7 was obtained.
Yield: 74.1 mg, yield ratio 80%.
Colorless oily substance; IR (neat) 1732 cm⁻¹; ¹H NMR (400MHz, CDCl₃) δ 3.68 (s, 3H) 3.66 (s, 3H), 3.17 (dd, 1H, J = 10.0, 8.8 Hz), 2.35 (m, 1H), 1.86-1.73 (m, 4H), 1.69 (ddd, 1H, J = 14.2, 4.4, 1.7 Hz), 1.48 (dd, 1H, J = 11.0, 8.8 Hz), 1.43-1.33 (m, 2H), 1.13 (s, 3H), 0.88 (s, 9H), 0.16 (s, 3H), 0.12 (s, 3H); ¹³C NMR (75 MHz, CDCl₃) δ · 176.4, 173.3, 76.6, 51.8, 51.0, 47.7, 41.3, 40.1, 35.4, 32.6, 26.0, 25.6, 24.9, 23.5, 18.2, -3.2, -3.5; LRMS m/z 313 (M-57)⁺; HRMS calcd for C₁₅H₂₅O₅Si: 313.1471, found: 313.1461.

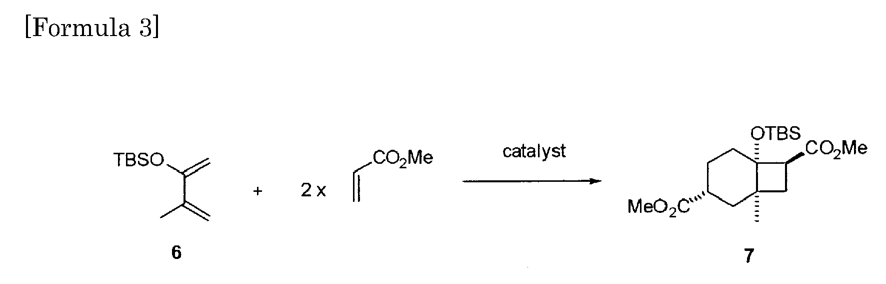

As is apparent from the working example described above, the polysubstituted bicyclo[4.2.0]octane compound was obtained at a satisfactory yield ratio from a diene compound and an alkene compound when bis(trifluoromethane)sulfonimide was used in the ratio of 5 mol% with respect to the starting materials.

All of the reactions in the working examples in the present specification can be performed in the same manner using solvents other than the "halogenated hydrocarbons" mentioned in the specification, e.g., solvents selected from esters and ethers.

### [Working Example 8]

An evaluation was conducted of growth inhibition of malaria parasites by a compound 9 derived by chemical conversion of cyclobutane compound 8 synthesized according to the present invention. As a result, the growth of cultured Plasmodium falciparum was 50% inhibited by a low concentration of 1 µM of compound 9.

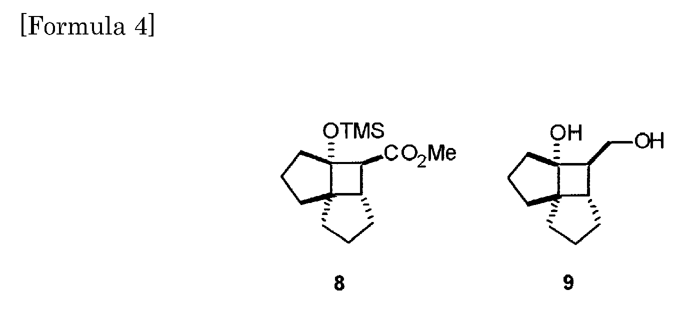

### [Working Example 9]

An evaluation was conducted of the anti-tumor effects of a compound 11 derived by chemical conversion of cyclobutane compound 10 synthesized according to the present invention. As a result, the growth of colon cancer cells (HCT116) was inhibited by a low concentration of 25 µg/mL of compound 11.

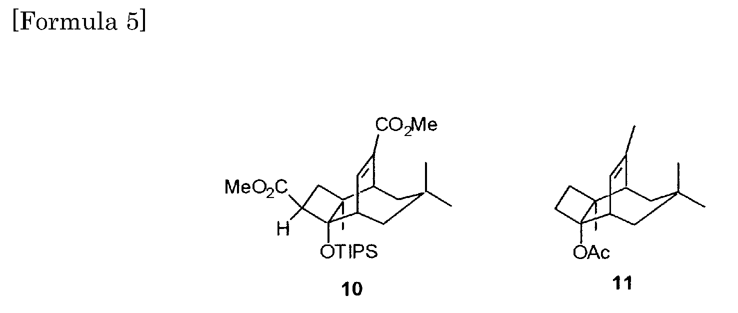

### INDUSTRIAL APPLICABILITY

The method of the present invention for manufacturing cyclobutane and cyclobutene compounds can be applied in the industrial synthesis of functional molecules, pharmaceutical products, and the like.

The cyclobutane and cyclobutene compounds manufactured by the present invention can be widely used as functional molecules, pharmaceutical products, agricultural chemicals, cosmetics, and flavorings, and as starting materials and synthesis intermediates for the same.

It is apparent that the present invention may be implemented in ways other than those mentioned in the description and specified in the working examples. Numerous improvements and modifications of the present invention can be made using the information contained in the description above, and are accordingly encompassed in the range of the attached claims.

## Claims

1. A method for manufacturing a polysubstituted cyclobutane compound indicated by Chemical Formula (3): 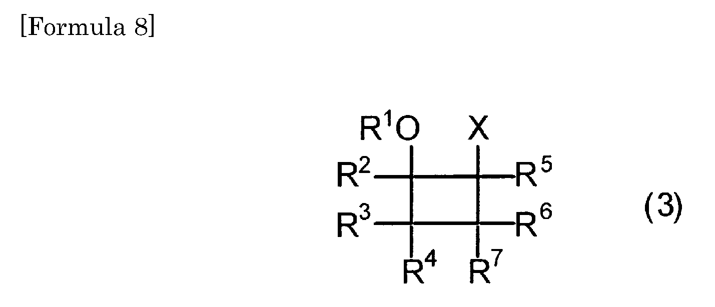 (wherein X and the substituents R¹ through R⁷ are the same as below), **characterized in that** a Brønsted acid catalyst is made to act in a non-aqueous solvent or without a solvent using as starting materials
an enol ether compound indicated by Chemical Formula (1): 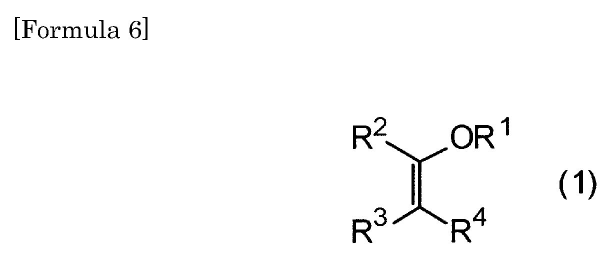 (wherein R¹ is an optionally substituted alkyl group, an optionally substituted aryl group, an optionally substituted heterocyclic group, or a silyl group; R², R³, and R⁴ are each independently a hydrogen atom, a halogen atom, an optionally substituted alkyl group, an optionally substituted aryl group, an optionally substituted heterocyclic group, or an optionally substituted alkyl alkoxy group, and may be the same or different; and the substituents of R¹ through R⁴ may be bonded to each other); and
an alkene compound in which a carbonyl group is substituted at the 1-position, wherein the alkene compound is indicated by Chemical Formula (2): 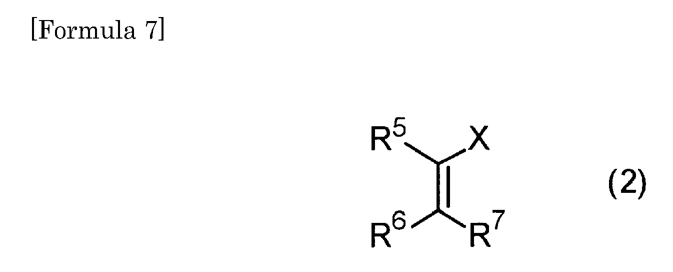 (wherein X is an ester carbonyl group, an amide carbonyl group, a ketocarbonyl group, or an aldehyde group; R⁵, R⁶, and R⁷ are each independently a hydrogen atom, a halogen atom, an optionally substituted alkyl group, an optionally substituted aryl group, or an optionally substituted heterocyclic group, and may be the same or different; and the substituents of X and R⁵ through R⁷ may be bonded to each other).

2. The method for manufacturing a polysubstituted cyclobutane compound of claim 1, **characterized in that** the enol ether compound is a silyl enol ether compound indicated by Chemical Formula (4): 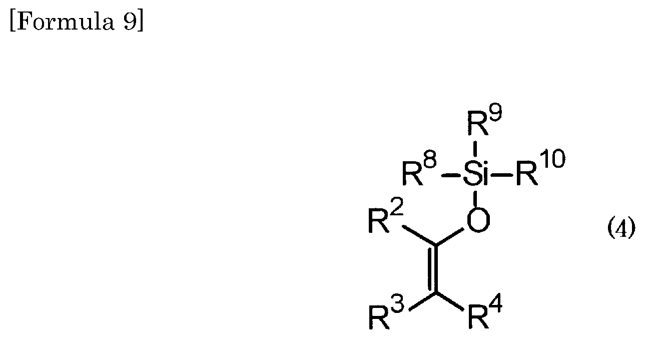 (wherein the substituents of R² through R⁴ are the same as above; R⁸, R⁹, and R¹⁰ are each independently a hydrogen atom, a halogen atom, an optionally substituted alkyl group, an optionally substituted aryl group, or an optionally substituted alkoxy group, and may be the same or different; and the substituents of R⁸ through R¹⁰ may be bonded to each other).

3. The method for manufacturing a polysubstituted cyclobutane compound of claim 1 or 2, **characterized in that** the alkene compound indicated by Chemical Formula (2) is an alkene compound having an ester carbonyl group indicated by Chemical Formula (5): 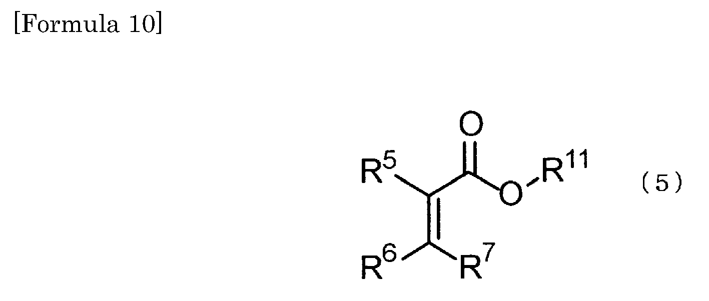 (wherein the substituents of R⁵ through R⁷ are the same as above; R¹¹ is an optionally substituted alkyl group, an optionally substituted aryl group, or an optionally substituted heterocyclic group; and the substituents of R⁵ through R⁷ may be bonded to each other).

4. The method for manufacturing a polysubstituted cyclobutane compound of any of claims 1 through 3, **characterized in that** the Brønsted acid catalyst is a polyfluoro-substituted sulfonyl compound indicated by Chemical Formula (6): 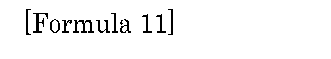 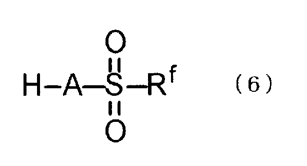 (wherein A is a nitrogen atom, an oxygen atom, a carbon atom, a sulfur atom, or a phosphorus atom that has a plurality of substituents or is unsubstituted as chemically allowed; and R^{f} is a heterocyclic group, an aryl group, or an alkyl group in which two or more fluorine atoms are substituted).

5. The method for manufacturing a polysubstituted cyclobutane compound of any of claims 1 through 4, **characterized in that** the polyfluoro-substituted sulfonyl compound is selected from the group consisting of a bis(polyfluoroalkyl)sulfonimide indicated by Chemical Formula (7), a bis(polyfluoroalkylsulfonyl)methane indicated by Chemical Formula (8), and a tris(polyfluoroalkylsulfonyl)methide indicated by Chemical formula (9): 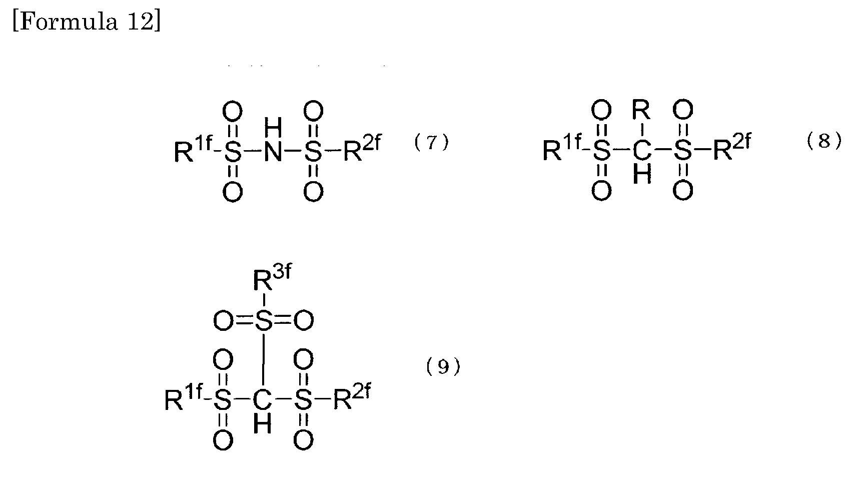 (wherein R^{1f}, R^{2f}, and R^{3f} are [each] independently a heterocyclic group, an aryl group, or an alkyl group in which two or more fluorine atoms are substituted; and R is a hydrogen atom or an organic group).

6. The method for manufacturing a polysubstituted cyclobutane compound of any of claims 1 through 5, **characterized in** selectively synthesizing a stereoisomer indicated by Chemical Formula (10) in which an ether substituent and a carbonyl side chain on a four-membered ring are in a trans relationship 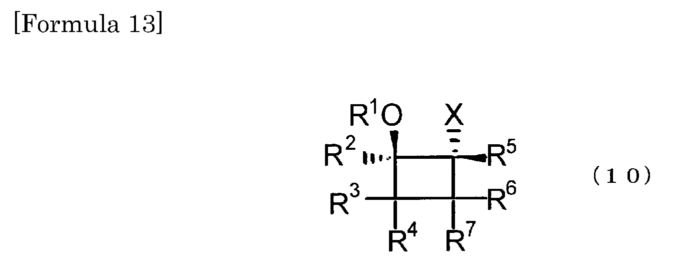 (wherein X and the substituents of R¹ through R⁷ are the same as above).

7. A polysubstituted cyclobutane compound synthesized by the method of any of claims 1 through 6.

8. A method for manufacturing a polysubstituted cyclobutene compound indicated by Chemical Formula (12): 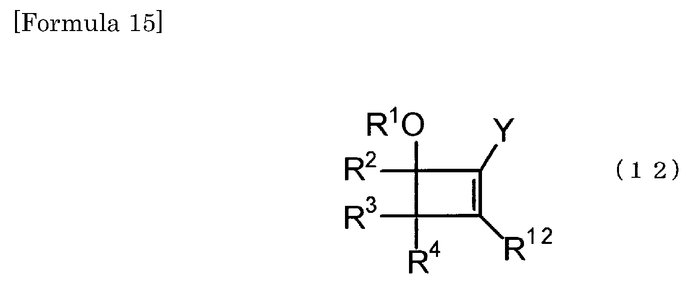 (wherein Y, R¹ through R⁴, and R¹² are the same as below), **characterized in that** a Brønsted acid catalyst is made to act in a non-aqueous solvent or without a solvent using as a starting material:
an enol ether compound indicated by Chemical Formula (1); and
an alkyne compound in which a carbonyl group is substituted at the 1-position, wherein the alkyne compound is indicated by Chemical Formula (11): 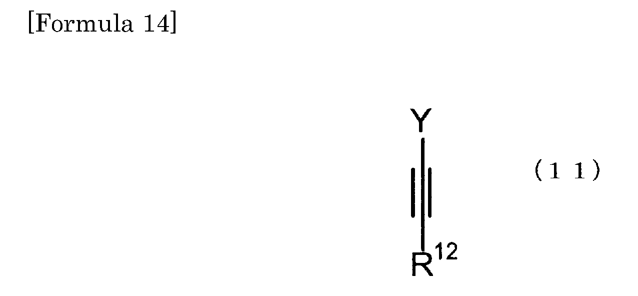
(wherein Y is an ester carbonyl group, an amide carbonyl group, a ketocarbonyl group, an aldehyde group, or a nitrile group; and R¹² is a hydrogen atom, a halogen atom, an optionally substituted alkyl group, an optionally substituted aryl group, or an optionally substituted heterocyclic group).

9. The method for manufacturing a polysubstituted cyclobutene compound of claim 8, **characterized in that** the enol ether compound is a silyl enol ether compound indicated by Chemical Formula (4).

10. The method for manufacturing a polysubstituted cyclobutene compound of claim 8 or 9, **characterized in that** the alkyne compound indicated by Chemical Formula (11) is an alkyne compound indicated by Chemical Formula (13): 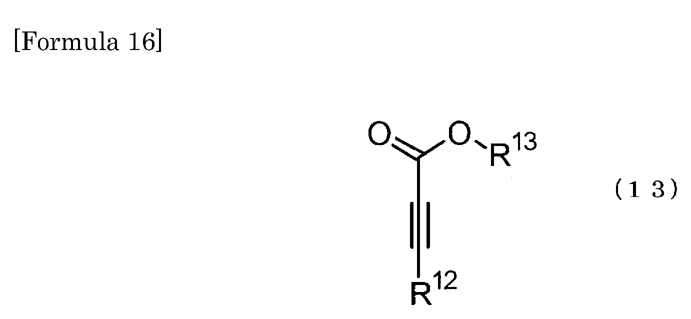 (wherein R¹² is the same as above; and R¹³ is an optionally substituted alkyl group, an optionally substituted aryl group, or an optionally substituted heterocyclic group).

11. The method for manufacturing a polysubstituted cyclobutene compound of any of claims 8 through 10, **characterized in that** the Brønsted acid catalyst is a polyfluoro-substituted sulfonyl compound indicated by Chemical Formula (6).

12. The method for manufacturing a polysubstituted cyclobutene compound of any of claims 8 through 11, **characterized in that** the polyfluoro-substituted sulfonyl compound is selected from the group consisting of a bis(polyfluoroalkyl)sulfonimide indicated by Chemical Formula (7), a bis(polyfluoroalkylsulfonyl)methane indicated by Chemical Formula (8), and a tris(polyfluoroalkylsulfonyl)methide indicated by Chemical Formula (9).

13. A polysubstituted cyclobutene compound manufactured by the manufacturing method of any of claims 8 through 12.

14. A method for manufacturing a compound having a bicyclo [4.2.0] octane main chain indicated by Chemical Formula (15): 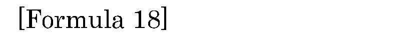 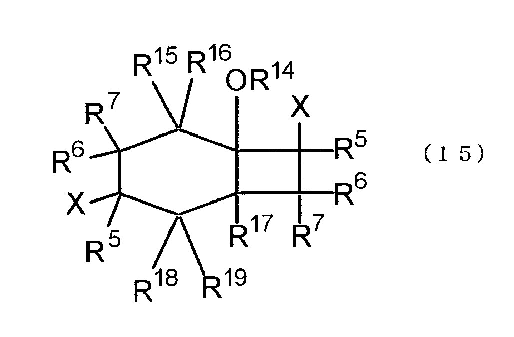 (wherein X, R⁵ through R⁷, and R¹⁴ through R¹⁹ are the same as below), **characterized in that** a Brønsted acid catalyst is made to act in a non-aqueous solvent or without a solvent using as a starting material:
an alkene compound indicated by Chemical Formula (2) in which a carbonyl group is substituted at the 1-position; and
a diene compound indicated by Chemical Formula (14): 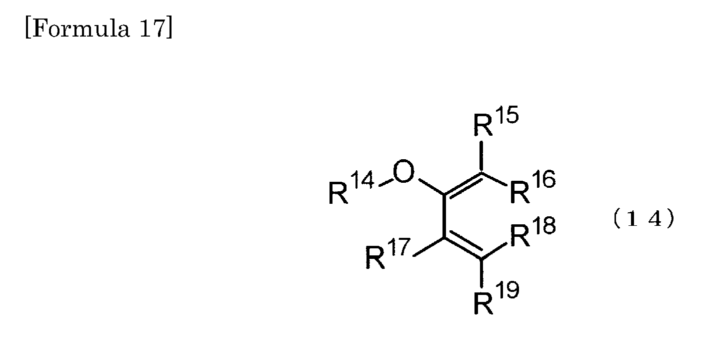
(wherein R¹⁴ is an optionally substituted alkyl group, an optionally substituted aryl group, an optionally substituted heterocyclic group, or a silyl group; R¹⁵ through R¹⁹ are each independently a hydrogen atom, a halogen atom, an optionally substituted alkyl group, an optionally substituted aryl group, an optionally substituted heterocyclic group, or an optionally substituted alkoxy group, and are the same or different; and the substituents of R¹⁴ through R¹⁹ may be bonded to each other).

15. The method for manufacturing a compound having a bicyclo[4.2.0]octane main chain of claim 14, **characterized in that** the Brønsted acid catalyst is a polyfluoro-substituted sulfonyl compound indicated by Chemical Formula (6).

16. The method for manufacturing a compound having a bicyclo[4.2.0]octane main chain of claim 14 or 15, **characterized in that** the polyfluoro-substituted sulfonyl compound is selected from the group consisting of a bis(polyfluoroalkyl)sulfonimide indicated by Chemical Formula (7), a bis(polyfluoroalkylsulfonyl)methane indicated by Chemical Formula (8), and a tris(polyfluoroalkylsulfonyl)methide indicated by Chemical Formula (9).

17. A compound having a bicyclo[4.2.0]octane main chain, manufactured by the manufacturing method of any of claims 14 through 16.

18. A compound having a bicyclo[4.2.0]octane main chain that is selected from the group consisting of a compound indicated by Chemical Formula (3), a compound indicated by Chemical Formula (12), and a compound indicated by Chemical Formula (15).
